(19) Europäisches Patentamt / European Patent Office / Office européen des brevets

(11) **EP 3 162 888 B1**

(12) **EUROPEAN PATENT SPECIFICATION**

(45) Date of publication and mention
of the grant of the patent:
**09.12.2020 Bulletin 2020/50**

(21) Application number: **15815209.0**

(22) Date of filing: **29.06.2015**

(51) Int Cl.:
**C12P 23/00** (2006.01)

(86) International application number:
**PCT/JP2015/068665**

(87) International publication number:
**WO 2016/002710 (07.01.2016 Gazette 2016/01)**

(54) **CAROTENOID-CONTAINING DRIED BACTERIAL CELL POWDER, AND METHOD FOR PRODUCING SAME**

CAROTINOIDHALTIGES GETROCKNETES BAKTERIENZELLENPULVER UND VERFAHREN ZUR HERSTELLUNG DAVON

POUDRE DE CELLULES BACTÉRIENNES SÉCHÉES CONTENANT DES CAROTÉNOÏDES, ET PROCÉDÉ POUR SA PRODUCTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.06.2014 JP 2014134830**

(43) Date of publication of application:
**03.05.2017 Bulletin 2017/18**

(73) Proprietor: **ENEOS Corporation
Chiyoda-ku
Tokyo 100-8162 (JP)**

(72) Inventors:
• **NAGAI Hidetada**
  **Tokyo 1008162 (JP)**
• **KAWASHIMA Yuki**
  **Tokyo 1008162 (JP)**
• **SUNADA Futoshi**
  **Tokyo 1008162 (JP)**
• **TAKAHASHI Toshiyuki**
  **Tokyo 1008162 (JP)**
• **AOYAGI Kenichi**
  **Tokyo 1008162 (JP)**

(74) Representative: **Altmann Stößel Dick
Patentanwälte PartG mbB
Dudenstrasse 46
68167 Mannheim (DE)**

(56) References cited:
EP-A1- 2 554 675          WO-A1-2011/122616
WO-A1-2012/114998     WO-A1-2016/194789
JP-A- H02 504 101        JP-A- H08 508 885
JP-A- 2002 223 787       JP-A- 2012 505 656
US-A1- 2013 330 298

• **GIL-HWAN AN* ET AL: "Improved Astaxanthin availability due to drying and rupturing of the red yeast, Xanthophyllomyces dendrorhous", FOOD SCIENCE AND BIOTECHNOLOGY, THE KOREA SOC. OF FOOD SCIENCE AND TECHNOLOGY, HEIDELBERG, vol. 15, no. 4, 1 January 2006 (2006-01-01), pages 506-510, XP008185578, ISSN: 1226-7708**
• **AN, G-H. ET AL.: 'Improved Astaxanthin availability due to drying and rupturing of the red yeast, Xanthophyllomyces dendrorhous' FOOD SCI. BIOTECHNOL. vol. 15, no. 4, 2006, pages 506 - 510, XP008185578**
• **YUKI KAWASHIMA ET AL.: 'Keiran Iroage ni Shiyo sareru Astaxanthin Gan'yu Kintai eno Kansoho no Eikyo' ABSTRACTS OF AUTUMN MEETING OF THE SOCIETY OF CHEMICAL ENGINEERS vol. 46 TH, 16 October 2014, XP008185596**
• **HIDETADA NAGAI: 'Shiryoyo Zeaxanthin no Kaihatsu' ENEOS TECHNICAL REVIEW vol. 56, no. 1, 25 February 2014, pages 23 - 27, XP008185594**

## Description

Technical Field

The present invention relates to a method for producing a dried bacterial cell powder containing a carotenoid, a dried bacterial cell powder produced thereby, and a method for producing feed for poultry .

Background Art

Carotenoid compounds have been added to feed (feedstuff) for the purpose of enhancing chicken egg yolk coloration or the red color of fish flesh (fish meat). Astaxanthin is a type of carotenoid used for enhancing the yolk color of poultry, as well as the color of fish or shellfish. When a feed (feedstuff) containing astaxanthin is used, the astaxanthin is absorbed *in vivo,* thereby enhancing the color of chicken egg yolk or fish flesh. Methods for producing astaxanthin include a method for extracting astaxanthin from shellfish, a method for culturing *Phaffia* yeast, a method for culturing a green alga, and organic synthesis methods. The genus *Paracoccus* is highly capable of producing astaxanthin, and therefore, dried bacterial cells thereof have been used for salmon feed (Non-Patent Literature 1).

Patent Literature 1 discloses a composition containing a carotenoid pigment having excellent preservation stability and a method for producing the same.

Patent Literature 2 discloses a method for producing astaxanthin using a microorganism of the genus *Brevundimonas.*

Patent Literature 3 discloses a method for producing yeast for feed using *Phaffia* yeast. EP 2 554 675 A1 discloses a method for manufacturing zeaxanthin by fermentation. EP 2 554 675 A1 shows that zeaxanthin can efficiently be produced by Paracoccus bacteria in a medium containing biotin without unwanted high gluconic acid or PHB. US 2013/330298 A1 discloses a zeaxanthin-enriched poultry egg, i.e. containing zeaxanthin in yolk at 3.5 mg to 10 mg per 100 g of yolk. An, G-H. et al. ("Improved Astaxanthin availability due to drying and rupturing of the red yeast, Xanthophyllomyces dendrorhous", FOOD SCI. BIOTECHNOL., vol. 15, no. 4, 2006, pages 506-510) discloses that astaxanthin can be prepared by drum-drying yeast on a hot (150°C) drum surface. However, An et al. also discloses that, although the cost of drum-drying was lower and the extractability was increased, an increased degradation of astaxanthin could be observed, making the method more difficult to use.

Citation List

Patent Literature

    Patent Literature 1: JP Patent Publication (Kokai) No. 2006-101721 A
    Patent Literature 2: JP Patent Publication (Kokai) No. 2006-340676 A
    Patent Literature 3: JP Patent Publication (Kokai) No. H8-182 A (1996)

Non-Patent Literature

Non-Patent Literature 1: Seibutsu-kogaku Kaishi (Bioengineering), 2010, vol. 88, no. 9, pp. 492-493.

Summary of Invention

Technical Problem

An object of the present invention is to provide a powder comprising a carotenoid for feed (foodstuff) having an improved color enhancing ability, and a method for producing the same.

Solution to Problem

As a result of intensive studies in order to achieve the above object, the present inventors found that a dried bacterial cell powder obtained by drum drying a carotenoid-producing microorganism is superior to that obtained by spray drying of the same with regard to its color enhancing ability when added to feed. This has led to the completion of the present invention.

That is, the present invention encompasses the following [1] to [13].

[1] A method for producing a dried bacterial cell powder containing astaxanthin , said method comprising the step of bringing bacterial cells of an astaxanthin -producing microorganism of the genus *Paracoccus* into contact with a heat transfer unit having a temperature of more than 100°C to carry out drying via heat transfer and the step of further pulverizing the dried bacterial cell powder for fine powderization, wherein the volume particle size (D50) of the dried bacterial cell powder is pulverized to 20 μm or less by the pulverization step.

[2] The production method according to [1], wherein the temperature of the heat transfer unit is equal to or exceeds 120°C.

[3] The production method according to any one of [1] to [2], wherein the drying is drying carried out by using a drum dryer, drying carried out by using a vacuum box dryer, drying carried out by using a multi-cylinder dryer, drying carried out by using a trough dryer, drying carried out by using a shelf dryer, or drying carried out by using a hot plate dryer.

[4] The production method according to any one of [1] to [3], wherein the produced powder is a powder for being added to a feed for poultry.

[5] A method for producing a feed for poultry, said method comprising the step of adding a dried bacterial cell powder produced by the method according to any one of [1] to [4] to a feed for poultry.

[6] A dried bacterial cell powder containing astaxanthin, produced by the method according to one of [1] to [6].

[8] Use of a dried *Paracoccus* bacterial cell powder containing astaxanthin, astaxanthin produced by the method according to any one of [1] to [4] or the dried bacterial cell powder of claim 6 for the manufacture of feed for poultry.

Advantageous Effects of Invention

According to the present invention, a dried bacterial cell powder having improved color enhancing ability can be produced. When such powder is added to a feed, the color of chicken egg yolk can be significantly enhanced compared to addition of a carotenoid-containing dried bacterial cell powder produced by conventional spray drying techniques. Further, according to the present invention, yolk color can be enhanced to an extent comparable to that possible with a feed comprising a dried bacterial cell powder containing a carotenoid produced by conventional spray drying techniques, even when less feed is used. This allows reduction of production costs.

Brief Description of the Drawings

Fig 1 shows a comparison of the efficacy of chicken egg yolk color enhancement.
Fig. 2 shows the results of ethanol extraction experiments.
Fig. 3 shows a semilog plot of (1-E) vs. *t* (a drum-dried product).
Fig. 4 shows a semilog plot of (1-E) vs. *t* (a spray-dried product).
Fig. 5 shows a semilog plot of (1-E) vs. *t* (a granulated spray-dried product).
Fig. 6 shows a comparison of the degree of dependence of the efficacy of chicken egg yolk color enhancement on dried bacterial cell particle size. Description of Embodiments

The present invention is described in further detail below. The scope of the present invention is not limited to the following descriptions, and therefore, the present invention may be appropriately modified also in embodiments other than the following embodiments without departing from the spirit of the present invention.

The present invention relates to a dried bacterial cell powder of a astaxanthin-producing microorganism of the genus *Paracoccus,* which has improved color enhancing abilities. Such dried bacterial cell powder can be produced by bringing bacterial cells of a astaxanthin-producing microorganism of the genus *Paracoccus* into contact with a heat transfer unit at a temperature exceeding 100°C for heat drying. More specifically, according to the present invention, a method for producing a dried bacterial cell powder containing a carotenoid is provided, said method comprising the step of bringing bacterial cells of a astaxanthin -producing microorganism of the genus *Paracoccus* into contact with a heat transfer unit having a temperature exceeding 100°C for heat drying.

Examples of suitable microorganisms to be used in the present invention include bacteria of the genus *Paracoccus,*. Examples of bacteria of the genus *Paracoccus* include *Paracoccus carotinifaciens, Paracoccus marcusii, Paracoccus haeundaensis, Paracoccus zeaxanthinifaciens, Paracoccus denitrificans, Paracoccus aminovorans, Paracoccus aminophilus, Paracoccus kourii, Paracoccus halodenitrificans* and *Paracoccus alcaliphilus.* However, the astaxanthin-producing microorganism of the genus *Paracoccus* used in the present invention is not limited to these examples.

In one embodiment, *Paracoccus carotinifaciens* can be used as a carotenoid-producing microorganism. One example of the strains of *Paracoccus carotinifaciens* is the *Paracoccus carotinifaciens* E-396 strain (FERM BP-4283).

Mutants of the astaxanthin-producing microorganism can also be used in the present invention. That is, a mutant strain having a modified ability to produce a carotenoid may be used in the present invention. Examples of such mutant include, but are not limited to, a strain that is highly capable of producing astaxanthin (JP Patent Publication (Kokai) No. 2001-95500 A), a strain that selectively produces canthaxanthin in a large amount (JP Patent Publication (Kokai) No. 2003-304875 A), a strain that selectively produces zeaxanthin and β-cryptoxanthin in large amounts (JP Patent Publication (Kokai) No. 2005-87097 A), and a strain that selectively produces lycopene (JP Patent Publication (Kokai) No. 2005-87100 A).

A mutant strain having a modified ability to produce a carotenoid can be obtained by, for example, mutagenesis treatment and screening. Examples of mutagenesis treatment include, but are not limited to, chemical methods using mutagens such as N-methyl-N'-nitro-N-nitrosoguanidine and ethyl methanesulfonate, physical methods involving, for example, ultraviolet or X-ray irradiation, and biological methods using gene recombination or a transposon.

Examples of methods of mutant screening include a method for selecting a mutant of interest based on the colony color on agar medium and a method for selecting a mutant of interest, which comprises culturing a mutant in a test tube, flask, fermenter, or the like and performing carotenoid pigment analysis by absorbance determination, high performance liquid chromatography (HPLC), thin layer chromatography (TLC), or the like. Mutation and selection may be performed once or a plurality of times.

Examples of a carotenoid produced by the carotenoid-producing microorganism of the present invention include, astaxanthin, β-carotene, lycopene, zeaxanthin, β-cryptoxanthin, canthaxanthin, phoenicoxanthin, adonixanthin, echinenone, asteroidenon, and 3-hydroxyechinenone.

A method for culturing a carotenoid-producing microorganism may be any method as long as the method is carried out under conditions that allow carotenoid production such as the following conditions. That is, a medium containing, for example, a carbon source, a nitrogen source, an inorganic salt, and optionally, a special necessary nutrient (e.g., a vitamin, amino acid, or nucleic acid), which are required for the growth of the carotenoid-producing microorganism, is used.

Examples of a carbon source include sugars such as glucose, sucrose, fructose, trehalose, mannose, mannitol, and maltose; organic acids such as acetic acid, fumaric acid, citric acid, propionic acid, malic acid, and malonic acid; alcohols such as ethanol, propanol, butanol, pentanol, hexanol, and isobutanol; and combinations thereof. The proportion of a carbon source to be added depends on the type thereof; however, it can in general be 1 to 100 g (e.g., 2 to 50 g) in 1 L of medium.

Examples of a nitrogen source include potassium nitrate, ammonium nitrate, ammonium chloride, ammonium sulfate, ammonium phosphate, ammonia, urea, and combinations thereof. The proportion of a nitrogen source to be added depends on the type thereof; however, it can in general be 0.1 to 20 g (e.g., 1 to 10 g) in 1 L of medium.

Examples of an inorganic salt include potassium dihydrogen phosphate, dipotassium hydrogen phosphate, disodium hydrogen phosphate, magnesium sulfate, magnesium chloride, iron sulfate, iron chloride, manganese sulfate, manganese chloride, zinc sulfate, zinc chloride, copper sulfate, calcium chloride, calcium carbonate, sodium carbonate, and combinations thereof. The proportion of an inorganic salt to be added depends on the type thereof; however, it can in general be 0.1 mg to 10 g in 1 L of medium.

Examples of special required nutrients include vitamins, nucleic acids, yeast extract, peptone, meat extract, malt extract, corn steep liquor, dried yeast, soybean cake, soybean oil, olive oil, corn oil, linseed oil, and combinations thereof. The proportion of the special required nutrient to be added depends on the type thereof and can generally be 0.01 mg to 100 g in 1 L of medium.

The pH of the medium is adjusted to, for example, pH 2 to 12 or pH 6 to 9.

It is possible to perform culture by shake culture or aeration culture at, for example, 10°C to 70°C (e.g., 20°C to 35°C) usually for 1 to 20 days (e.g., 2 to 9 days). The carotenoid-producing microorganism is cultured under such conditions. As a result of culture, bacterial cells of the microorganism intracellularly and/or extracellularly produce a large amount of a carotenoid.

A culture solution obtained by the above culture method can be appropriately concentrated. Examples of concentration methods include membrane concentration and centrifugation.

Following the aforementioned step, medium components can be removed. Upon centrifugation, if concentration was carried out, then water can be added to the resulting concentrated liquid so as to remove medium components. If membrane separation is employed, diafiltration is performed so as to remove medium components. The amount of water added may be approximately 1 to 5 times that of the concentrated liquid, although such amount would vary depending on the pigment content or the like in the concentrated liquid.

Further, a culture product or a concentrate is dried in order to obtain a dried bacterial cell powder. That is, according to the present invention, the astaxanthin-containing bacterial cells of the genus *Paracoccus* obtained in the form of a culture product or bacterial cell slurry are dried to form a powder. The types of drying can be roughly divided into hot-air heat transfer and conductive heat transfer. A dryer with hot-air heat transfer mechanism caries out drying by applying hot air on the object of interest. A dryer with conductive heat transfer mechanism caries out drying by conductive heat transfer via a heated transfer unit (i.e., a plate) thereby applying heat to the object of interest.

Examples of dryers with a hot-air heat transfer mechanism include a tunnel dryer, an impinging jet dryer, a band dryer, a rotary dryer, a fluid bed dryer, a spray dryer, and an airflow dryer. A spray dryer is intended to spray a liquid or a slurry into hot air so as to obtain a fine particle product. When hot air is applied to a liquid or a slurry, the medium thereof (e.g., water) evaporates, and thus, dried particles or powder can be obtained. Droplets generated by an atomizer vanish during drying via the heated air supply. The temperature of the gas used for drying is, in general, high. If, however, the medium of the liquid or slurry to be dried is water, the liquid surface temperature of sprayed spherical droplets never actually exceeds 100°C, at which temperature water evaporates, during water desorption. It therefore is believed that the rate of heat supply from the heated air cannot be increased significantly. In the case of spray drying, drying begins to take place on the droplet surfaces, which makes the pores on the surface of the relevant object shrink. This could cause surface hardening.

Examples of dryers with a conductive heat transfer mechanism include a vacuum box dryer, a drum dryer (also referred to as a drum-type dryer), a multi-cylinder dryer, a paddle dryer, a shelf dryer, and a hot plate dryer. In a dryer with a conductive heat transfer mechanism, the object of interest is brought into contact with a heated heat transfer unit. This means that when the temperature of the heat transfer unit exceeds 100°C, a very large amount of heat is supplied due to high efficiency of heat transfer, which may allow the water desorption rate (dispersion rate) to increase. In order to bring the object of interest into contact with the heat transfer unit, the object may be applied to the heat transfer unit by coating or dripping. The heat transfer unit may be formed with a material such as a metal or a non-metal material (e.g., acrylic resin) which is heated by thermal vibration. The heat transfer unit may be in the form of a plate having an appropriate shape, such as a flat, curved, or semi-cylinder shape, as long as it has a sufficient heat transfer area. Once the object of interest is dried after being brought into contact with the heat transfer unit, it forms a sheet shape (i.e., a membrane shape), and thus, can be removed using a scraper or the like where necessary.

The period of time during which the heat transfer unit is brought into contact with the object to be dried may be, but not limited to, in general, several minutes or less, e.g., 5 minutes or less, 4 minutes or less, 3 minutes or less, 2 minutes or less, or .g., 60 seconds or less. The material of the heat transfer unit may be heated under ordinary pressure, under increased or decreased pressure, or *in vacuo*. The temperature of the heat transfer unit must exceed 100°C. In one embodiment, the temperature of the heat transfer unit exceeds 110°C, 120°C, 130°C, or 140°C. In another embodiment, the temperature of the heat transfer unit is 220°C or less, 210°C or less, 200°C or less, 190°C or less, 180°C or less, 170°C or less, 160°C or less, or 150°C or less. In yet another embodiment, the temperature of the heat transfer unit is 140°C to 160°C (e.g., 140°C to 150°C).

When a drum dryer is used, the object of interest having liquid or paste form is applied to the surface of a heated drum and the object of interest is dried by rotating the drum. There are two types of drum dryers, namely, double drum dryers and single drum dryers. Examples of double drum dryers include an internal rotation type dryers, external rotation type dryers, and vacuum dryers. Examples of single drum dryers include top feed type dryers, dip feed type dryers, and splash feed type drum dryers. Regardless of the type, the obtained dried product is a flake or sheet form.

A drum dryer may be operated under arbitrary conditions as long as a dried product can be obtained. For example, the drum rotation speed may be, but not limited to, 1 to 5 rpm, 2 to 5 rpm, 2 to 4 rpm, or 2 to 3 rpm. The contact time and the heat transfer unit temperature are as defined above.

A dried product that has been formed into flakes or a sheet as a result of drying via heat transfer, such as via drum drying, can after drying be powdered in a step of crushing using a hammer mill or the like. The resulting product is referred to herein as a "crushed product" in some cases for the sake of convenience. Next, a dried product or a crushed product thereof can be even more finely pulverized in a Jet Mill step. The resulting product is herein referred to as a "pulverized product" or a "finely powderized product" in some cases for the sake of convenience. Such crushed or pulverized product is obtained by breaking up a sheet-shaped dried product into pieces, which means that the product retains its planar shape when broken into small pieces. The term "dried bacterial cell powder" used herein refers not only to a bacterial cell powder obtained as a result of drying via heat transfer but also to a finely powderized product thereof in some cases.

In one embodiment, a dried bacterial cell powder obtained by drying, e.g., drum drying, via heat transfer according to the present invention can be finely powderized by Jet Mill crushing. This crushing step allows the powder to have a particle size of, for example, 1 $\mu$m to 30 $\mu$m, 1 $\mu$m to 20 $\mu$m, 5 $\mu$m to 20 $\mu$m, or 7 $\mu$m to 20 $\mu$m. The term "particle size" used herein means a diameter of a particle regarded as having a spherical shape. In addition, the term "average particle size" used herein means a volume particle size (D50). The term "volume particle size (D50)" is also referred to as "median size" that is a particle size at which the cumulative volume becomes 50% upon determination of particle size distribution. The particle size can be determined by a known technique such as laser diffraction/scattering analysis.

According to the present invention, the dried bacterial cell powder or a finely powderized product thereof can be added to feed (feedstuff) for poultry farming. Examples of poultry include chickens, quails, turkeys, guineafowls, pigeons, ducks, and geese, with chickens being particularly preferable. For instance, 1 g of dried bacterial cells of a bacterium of the genus *Paracoccus* contains approximately 2.1 mg to 2.5 mg of astaxanthin. Accordingly, the use of the dried bacterial cells of the present invention for a feed allows the carotenoid concentration, and in particular, the astaxanthin concentration in yolk of eggs laid by poultry fed with the feed to increase, resulting in color enhancement.

In one embodiment of the present invention, a method for producing a feed for poultry, comprising a step of adding a dried bacterial cell powder of a astaxanthin-producing microorganism of the genus *Paracoccus* produced by the method according to the present invention is provided. Poultry is fed with such feed so as to be raised for egg collection such that eggs with enhanced yolk color can be obtained. A dried bacterial cell powder of a carotenoid-producing microorganism is added to a feed for poultry farming such that 100 g of the feed contains 0.1 mg or more, 0.4 mg or more, 0.8 mg or more, or 1 mg or more (e.g., 0.4 to 50 mg or 0.4 to 20 mg) of astaxanthin produced in the carotenoid-producing microorganism. The dried bacterial cell powder can be mixed with a feed used for poultry farming. Although the content of the dried bacterial cell powder in the feed for poultry farming will vary depending on the content of astaxanthin in the dried bacterial cells, it can be preferably 0.01% by weight or more and 0.05% by weight or more (e.g., 0.01% to 5% by weight).

The dried bacterial cell powder of the present invention can be mixed with a feed for feeding poultry. It also may be mixed into a premix together with vitamins and the like in advance for feeding.

A variety of carotenoid compounds contribute to the yolk color. Although astaxanthin is specifically mentioned as a representative carotenoid in some embodiments for explanation of the present invention, the color enhancing ability of the present invention is not particularly limited to that of astaxanthin and a variety of carotenoid compounds, including metabolites of astaxanthin contained in cells of carotenoid-producing microorganisms such as *Paracoccus* bacterial cells and astaxanthin precursors, also contribute to the overall color enhancing effect of the present invention.

Poultry fed with a feed containing the dried bacterial cell powder of the present invention lay eggs enriched with a carotenoid. According to the method of the present invention, poultry eggs with color-enhanced yolk containing 0.1 mg or more, 0.2 mg or more, or 0.3 mg or more (e.g., 0.2 mg to 10 mg or 0.2 mg to 5 mg) of astaxanthin (per, for example, 100 g of yolk) can be obtained.

The dried bacterial cell powder of the present invention is obtained by drying (e.g., drum drying) based on the conductive heat transfer mechanism but not by drying (e.g., spray drying) based on the hot-air heat transfer mechanism (for spray drying). A dried bacterial cell powder obtained by spray drying may be referred to herein as a "spray-dried product." Further, a dried bacterial cell powder obtained via drum drying may be referred to as a "drum-dried product." Further, a product obtained via Jet Mill crushing of a drum-dried product may be referred to as a "finely pulverized drum-dried product." Dried bacterial cell powder according to the present invention obtained by drying via heat transfer differs significantly from a spray-dried product not only in terms of shape but also in terms of physical properties, reflecting differences of the type of drying.

Since the drum-dried product and the finely pulverized product of the present invention have a sheet shape and are dried at a high temperature exceeding 100°C, it is believed that this may allow astaxanthin contained in the dried powder to readily diffuse from the powder so as to be absorbed *in vivo.* On the other hand, a spray-dried product predominantly has particles of spherical shape and is rapidly dried over a short period of time. It therefore is believed that spray-drying causes pores of the particle surface to shrink rapidly, this in turn leading to surface hardening. As such, it is believed that, regarding conventional spray-dried products, this structure becomes an obstacle for astaxanthin diffusion, thereby rendering it relatively difficult for *in vivo* absorption of the astaxanthin contained therein.

The above differences can be evaluated by, for example, subjecting astaxanthin contained in the dried bacterial cell powder dried via heat transfer according to the present invention, e.g., a drum-dried product, to solvent extraction (e.g., ethanol extraction). Further, the astaxanthin extraction rate of the dried bacterial cell powder dried via conductive heat transfer, drum-dried product or finely pulverized product according to the present invention, when subjected to ethanol extraction can be compared with the astaxanthin extraction rate of a spray-dried product. While it is known that the solvent extraction rate depends on temperature, it is possible to determine whether or not there is a special obstacle in the pathway of carotenoid diffusion from a dried product based on the temperature-dependency relationship.

The following is a description of the change of the diffusion coefficient D of astaxanthin upon ethanol extraction which can be represented by the quotient ($b_{25}/b_{35}$) as the result of dividing the diffusion coefficient D when determined at 25°C by the diffusion coefficient D when determined at 35°C according to the present invention.

The rate of extraction of astaxanthin from a dried bacterial cell powder with the use of ethanol is expressed as $E = C/C_e$, where C represents the concentration of an astaxanthin extract in an external solution (ethanol) (A480), and $C_e$ represents the equilibrium concentration.

The process of diffusion/desorption at a constant surface concentration is expressed by the following series formula, which is applicable when diffusion is constant.

[Mathematical Formula 1]

$$1 - E = 1 - \frac{\overline{c_s}}{c_{s0}} = 1 - \frac{8}{\pi^2} \sum_{n=0}^{\infty} \frac{1}{(2n+1)^2} exp\left[-\frac{(2n+1)^2 \pi^2}{4R^2} Dt\right] \qquad (1)$$

In formula (1) above, D represents the diffusion coefficient within the solid of the material being subjected to extraction, and R represents the particle radius of material being subjected to extraction. In addition, the concentration in the solid phase is designated as "Cs," and the average concentration thereof is designated as follows:

[Mathematical Formula 2]

$$\bar{C}_S$$

The initial concentration (equivalent to the total extraction concentration) is designated as "Cso." If the external medium is sufficiently large (abundant) and the final equilibrium concentration $C_e$ is low, then the assumption that the surface concentration is constant is sufficiently satisfied. If a semilog plot (single logarithmic plot) of (1-E) vs. dimensionless time ($tD/R_2$) is created, a linear line is obtained when (1-E) < 0.8. This is because only the first term of the series term in formula (1) becomes dominant, which results in the following formula (2).

[Mathematical Formula 3]

$$1 - E = 1 - \frac{C_S}{C_{S0}} = 1 - \frac{C}{C_e} \approx \frac{8}{\pi^2} exp\left[-\frac{\pi^2}{4}\frac{Dt}{R^2}\right] \qquad (2)$$

Accordingly, diffusion coefficient D can be determined based on the slope of the semilog plot of (1-E) vs. *t*. The slope of the semilog plot of (1-E) vs. *t* is designated herein with the letter "b," the value of "b" at 25°C is designated as "$b_{25}$" and the value of "b" at 35°C is designated as "$b_{35}$", for sake of convenience.

In the present specification, analysis is carried out by assuming that the above slope value corresponds to the overall mass transfer coefficient ($K_S = D/R^2$). Since $K_S$ is proportional to diffusion coefficient D, even if the temperature varies, it is believed that $K_S$ can be extrapolated based on the temperature dependency of the diffusion coefficient alone if the system is diffusion-dominant.

The following relationship holds between the diffusion coefficient D for diffusion in a solution and the absolute temperature T:

$$D_\eta/T = Constant$$

[where $\eta$ represents the viscosity of the solution (i.e., a solvent in a diluted solution)].

Under ideal conditions, $\eta = 0.914$ mPa·s (35°C, 308K) and $\eta = 1.096$ mPa·s (25°C, 298K) and, therefore, the calculated quotient $b_{25}/b_{35}$, derived from the relationship "$D_\eta/T = Constant$", is (0.914/308)/(1.096/298) = 0.807.

That is, in the present specification,
if the extraction rate is expressed as E = C/$C_e$
[wherein C represents the astaxanthin extract concentration in ethanol, and $C_e$ represents the equilibrium concentration], the value of the slope of the semilog plot of (1-E) vs. time (t) is designated with the letter "b,"
the value of "b" at 25°C is designated as "$b_{25}$" and
the value of "b" at 35°C is designated as "$b_{35}$",
then the temperature change ratio ($b_{25}/b_{35}$) at 25°C and at 35°C regarding the astaxanthin diffusion coefficient D upon ethanol extraction is a ratio which can be derived from the following relationship:

$$"D_\eta/T = Constant"$$

[wherein D represents the diffusion coefficient, $\eta$ represents the solution viscosity, and T represents the absolute temperature]. The quotient $b_{25}/b_{35}$ becomes close to 0.807 for a given test sample when there is no special obstacle (i.e., resistance) regarding the diffusion coefficient upon extraction.

In one embodiment, the dried *Paracoccus* bacterial cell powder containing astaxanthin of the present invention has a temperature change ratio ($b_{25}/b_{35}$) at 25°C and at 35°C, regarding the astaxanthin diffusion coefficient D upon ethanol extraction, of 0.807 ± 0.05, for example, 0.807 ± 0.04, 0.807 ± 0.03, 0.807 ± 0.02, 0.807 ± 0.01, 0.807 ± 0.005, 0.807 ± 0.004, 0.807 ± 0.003, 0.807 ± 0.002 (i.e., 0.805 to 0.809 or 0.807 to 0.809), when the volume particle size (D50) of said powder is 7 to 12 μm.

The present invention is described with reference to the Examples below. The scope of the present invention is not limited to the Examples.

Examples

Carotenoid quantities were determined via high performance liquid chromatography (HPLC) as described below.

Two columns (Inertsil SIL-100A, 5 μm (φ 4.6 × 250 mm), GL Sciences) were connected in series for use. Elution was carried out by applying an n-hexane/tetrahydrofuran/methanol liquid mixture (40:20:1) serving as a mobile phase at a constant temperature (that is, at roughly room temperature) and at a rate of 1.0 mL per minute. During measurements, samples were each dissolved in tetrahydrofuran and appropriately diluted with the mobile phase such that injection volume was adjusted to 20 μL. Detection of the column eluate was carried out at a wavelength of 470 nm. In addition, Sigma-Aldrich's astaxanthin (Cat. No. A9335) was used as a standard product for quantitative determination. The quantity of astaxanthin to be used as a standard product was determined by calculating the absorbance (477 nm) using a spectrophotometer and the area proportion (corresponding to purity) via HPLC (470 nm). The absorbance at 477 nm (A) and the astaxanthin peak area percentage (%) (B) upon HPLC analysis under the above conditions were determined for the standard solution. Then, the astaxanthin concentration was calculated using the following formula.

[Mathematical Formula 4]

$$\text{Astaxanthin concentration (mg/L)} = A \div 2150 \times B \times 100$$

The specific absorption coefficient of astaxanthin in the n-hexane/tetrahydrofuran/methanol liquid mixture (40:20:1) was as described below.

[Mathematical Formula 5]

$$E^{1\%}_{1\ cm} \text{ value (477 nm)} = 2150$$

The average particle size (i.e., volume particle size D50) was determined using a light-scattering particle size distribution analyzer (Seishin Enterprise Co., Ltd., LMS-2000e) in the Examples.

Example 1: Chicken egg yolk color enhancement (differences resulting from the different types of drying)

A mutant obtained via nitrosoguanidine mutagenesis of the *Paracoccus carotinifaciens* E-396 strain (FERM BP-4283) was used as a *Paracoccus* bacterial strain capable of producing a carotenoid. The strain was cultured in a medium for flask seeding and then in a main culture medium at 28°C under aerobic conditions until the bacterial cell concentration had reached the maximum level. Next, the resulting cells were harvested using a centrifuge and collected.

In general, a dried powder obtained through spray drying is a fine particle powder. On the other hand, drum drying results in a dried powder with a relatively large particle size. Therefore, in order to compare a spray-dried product with a drum-dried product, a granulated spray-dried product was used herein. The granulated spray-dried product was produced by directly drying the collected *Paracoccus* bacterial cells using a granulating type spray dryer. The granulated spray-dried product had an average particle size (i.e., volume particle size D50) of 385 μm.

A drum-dried product was produced by directly drying the collected *Paracoccus* bacterial cells using a double drum dryer under the following operating conditions: frequency of drum rotation: 3.5 rpm; and drum temperature: 140°C. The drum-dried product had an average particle size (i.e., volume particle size D50) of approximately 100 to 125 μm.

The drum-dried product was further finely powderized (i.e., pulverized) using a Jet Mill (Seishin Enterprise Co., Ltd.) so that the average particle size (D50) was adjusted to 9 μm. The resultant was designated as a finely pulverized drum-dried product.

Each of the dried bacterial cell products was added to a basal feed (Layer S7, Chubushiryo Co., Ltd.) so that the astaxanthin concentration in the feed was adjusted to approximately 0.8 mg/100 g.

Laying hens were divided into 3 groups (of 3 individuals each) as follows: a group to be fed using a granulated spray-dried product; a group to be fed using a drum-dried product; and a group to be fed using a finely pulverized drum-dried product (average particle size D50: 9 μm). They were then raised for 4 weeks. The feed dosage was adjusted to 100 g/day.

After 4 weeks of feeding, eggs were collected from each experimental group. The obtained chicken egg yolk astaxanthin content was determined via high performance liquid chromatography.

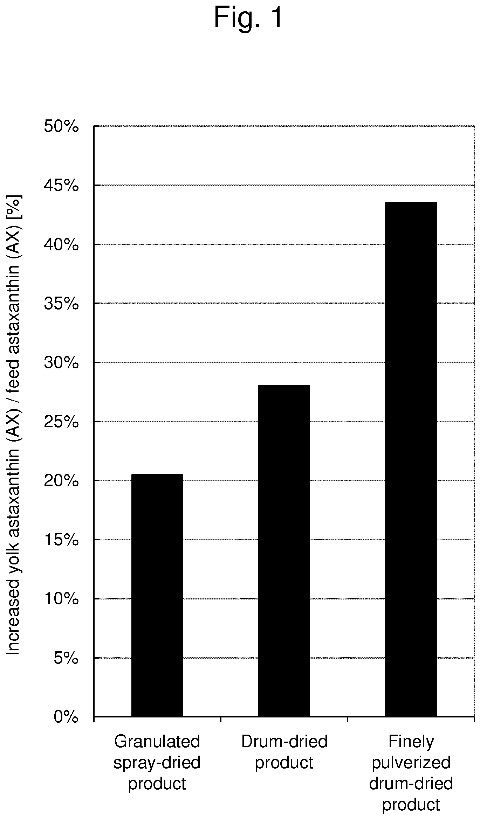

Results are summarized in Fig. 1. Fig. 1 shows an increase in the content of astaxanthin in chicken egg yolk at an astaxanthin concentration of approximately 0.8 mg/100 g in the feed. The amount of astaxanthin transferred into the yolk was greater (larger) for the drum-dried product and the pulverized drum-dried product than that for the granulated spray-dried product.

Example 2: Clarification of physical properties of dried bacterial cells via ethanol extraction

(Experimental method)

Samples (materials)

A: Finely pulverized product (finely pulverized drum-dried product)
B: Drum-dried product
C: Spray-dried product
D: Granulated spray-dried product

Samples A to D were produced in the manner described in Example 1. The finely pulverized drum-dried product (A) had a volume particle size D50 of 9 $\mu$m. The drum-dried product (B) had a volume particle size D50 of 125 $\mu$m.

Extraction solvent:

Ethanol (99.5%) ($C_2H_5OH$ (molecular weight: 46.07), Wako Pure Chemical Industries, Ltd. (Cat. No. 052-03343))
Short-time extraction behaviors were analyzed by experimentation using microplates.

Procedures

i) Sample solutions A to D each having an astaxanthin concentration of 1 mg (dried bacterial cell weight)/mL (EtOH) were prepared (2 mL per well in a multiwell cell capture plate 353047 (Falcon) with 24 wells).
ii) Each plate was shaken using a shake incubator (S1-300C) (25°C, 300 rpm).
iii) Sampling was initiated 3 minutes after the start of shaking, from which time the supernatant was collected at 5-minute intervals, followed by filtration through a 0.45-$\mu$m filter.
iv) Each sample was diluted twofold with the addition of ethanol (the sample (0.1 mL) + 100% EtOH (0.1 mL)), and the absorbance of each sample was determined (at a final volume 0.2 mL per well). After the sample volume had been set to 0.2 mL per well in a 24-well plate, the droplet in each well formed a lens-like shape. The thickness of such droplet was considered equivalent to the optical path length to be measured.

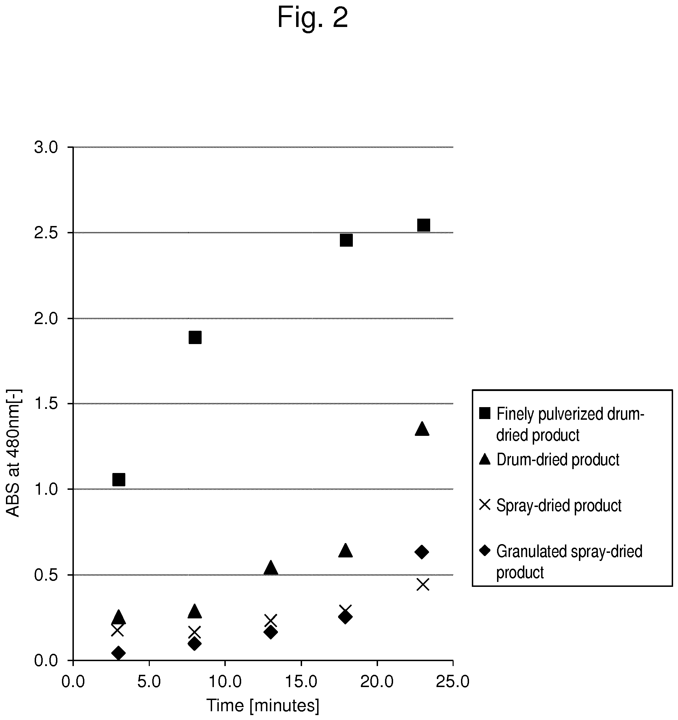

Results are shown in Fig. 2. The order of extraction rates for up to 18 minutes was as follows: (A) finely pulverized product > (B) drum-dried product > (C) spray-dried product > (D) granulated spray-dried product.

Determination of temperature dependence of ethanol extraction and evaluation of extraction characteristics

The average amount of astaxanthin extracted (determined at A480) with respect to the amount of dried bacterial cells was designated as the extraction equilibrium value. Extraction experiments were carried out using light-proof bottles at 25°C and 35°C.

Procedures

i) Sample solutions A to D each having an astaxanthin concentration of 0.1 mg (dried bacterial cell weight)/mL (EtOH) were prepared (30 mL each in a light-proof bottle).
ii) Each bottle was shaken in a thermostatic bath (EYELA NTT-2000, SS1000) (speed controller dial 3). In this case, the solvent was allowed to reach the measurement temperature prior to measurement, and then the experiment was initiated.

 iii) The supernatant was collected from each sample at predetermined time intervals. Sampling was initiated 5 minutes after the start of shaking.

iv) Each collected sample was diluted with ethanol (0.1 mL of the sample + 0.2 mL of 100% EtOH). The absorbance of each sample was determined (0.2 mL per well of a 24-well plate).

The extraction rate was expressed as E = $C/C_e$, where C represents the concentration of an extract in an external solution (ethanol) (A480), and $C_e$ represents the equilibrium concentration (corresponding to the A480 value of sample B after the elapse of 21 hours at 35°C).

Analysis by a diffusion model

The process of diffusion/desorption at a constant surface concentration is expressed by the following series formula, which is applicable when diffusion is constant.
[Mathematical Formula 6]

$$1 - E = 1 - \frac{\overline{c_S}}{c_{S0}} = 1 - \frac{8}{\pi^2} \sum_{n=0}^{\infty} \frac{1}{(2n+1)^2} exp\left[-\frac{(2n+1)^2 \pi^2}{4R^2} Dt\right] \qquad (1)$$

In formula (1) above, D represents the diffusion coefficient within the solid of the material being subjected to extraction, and R represents the particle radius of the material being subjected to extraction. The concentration within the solid phase is designated as "$C_S$," and the average concentration thereof is designated as follows:

[Mathematical Formula 7]

$$\overline{C_S}$$

The initial concentration (equivalent to the total extraction concentration) is designated as "$C_{S0}$." If the external medium is sufficiently abundant (large) and the final equilibrium concentration $C_e$ is low, then the assumption that the surface concentration is constant is sufficiently satisfied. If a semilog plot of (1-E) vs. dimensionless time ($tD/R_2$) is created, then a linear line can be obtained when (1-E) < 0.8. This is because only the first term of the series term in formula (1) becomes dominant, resulting in the following formula (2).
[Mathematical Formula 8]

$$1 - E = 1 - \frac{c_S}{c_{S0}} = 1 - \frac{C}{C_e} \approx \frac{8}{\pi^2} exp\left[-\frac{\pi^2}{4} \frac{Dt}{R^2}\right] \qquad (2)$$

Accordingly, diffusion coefficient D can be determined based on the slope of the semilog plot of (1-E) vs. *t*. The slope of the semilog plot of (1-E) vs. *t* is designated with the letter "b," the value of "b" at 25°C is designated as "$b_{25}$" and the value of "b" at 35°C is designated as "$b_{35}$" for the sake of convenience.

In this experimental system, analysis is carried out by regarding the above slope value as corresponding to the overall mass transfer coefficient ($K_S = D/R^2$). Since $K_S$ is proportional to the diffusion coefficient D, even if the temperature varies, it is believed that $K_S$ can be extrapolated based on the temperature dependency of the diffusion coefficient alone if the system is diffusion-dominant.

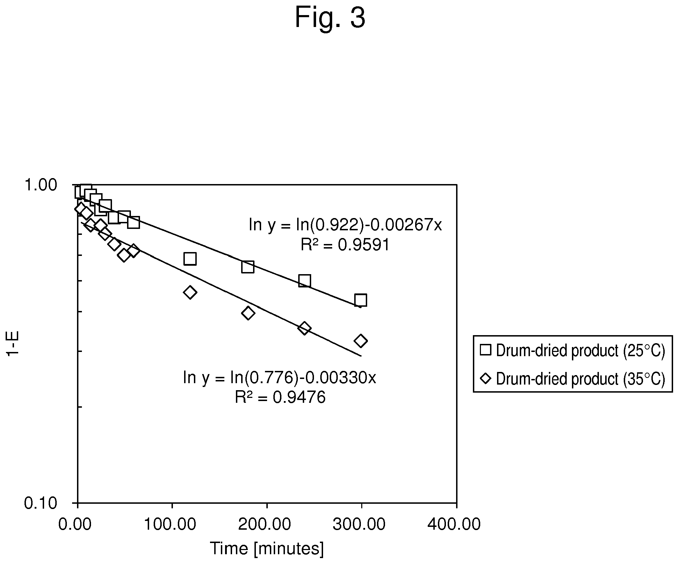

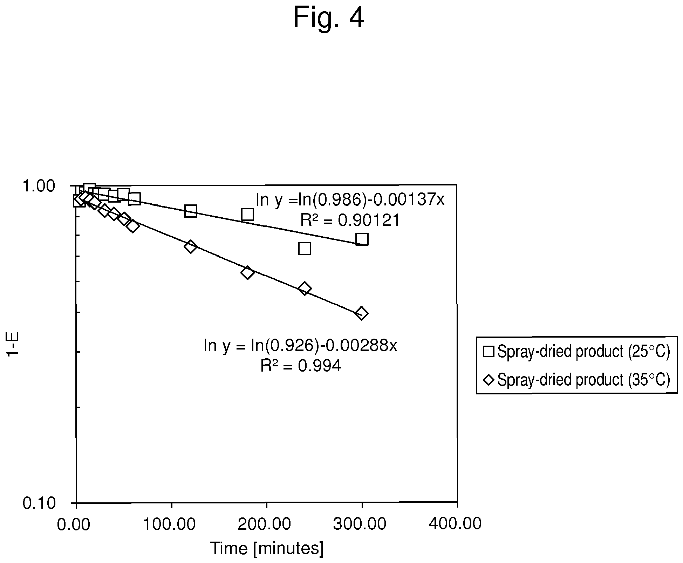

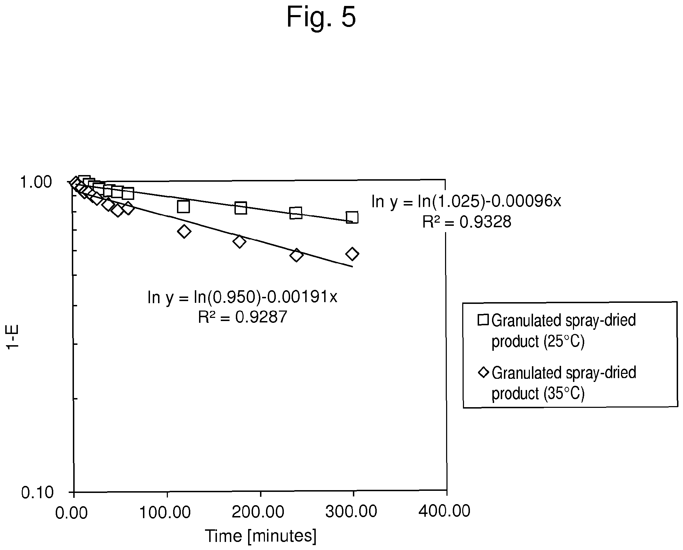

Figs. 3 to 5 show the results of the semilog plot of (1-E) vs. *t* in the sample extraction experiment. The figures show the presence of regions that can be linearly approximated and results of the least-square method are shown in the figures. The diffusion coefficient D for diffusion in a solution follows the relationship below with regard to absolute temperature T:

$$D_\eta/T = Constant$$

[where $\eta$ represents the viscosity of the solution (i.e., a solvent in a diluted solution)].

Since $\eta$ = 0.914 mPa·s (35°C, 308K) and $\eta$ = 1.096 mPa·s (25°C, 298K), the ratio $b_{25}/b_{35}$ of the slopes, derived from the relationship "$D_\eta/T$ = Constant", is calculated as (0.914/308)/(1.096/298) = 0.807. In Fig. 3, the quotient resulting from the division of one slope value by the other (i.e., $b_{25}/b_{35}$) is 0.00267/0.00330 = 0.809, indicating that the measured value approximately corresponds to the calculated value. This relationship holds when there is no special obstacle (i.e., resistance) regarding the diffusion coefficient upon extraction.

The above relationship does not necessarily hold for the other samples (spray-dried product C and granulated spray-dried product D). In addition, the slope itself represents the extraction rate and if the temperature increases, the extraction rate increases and the increase is in the following order: drum-dried product B > spray-dried product C > granulated spray-dried product D. For the finely pulverized drum-dried product (A), the extraction rate was extremely fast, and data varied greatly and, therefore, the finely pulverized drum-dried product (A) was not subjected to analysis to calculate the ratio of slopes (the quotient resulting from dividing one slope value by another). However, the quotient resulting from dividing one slope value by the other (i.e., $b_{25}/b_{35}$) for the drum-dried product (B) is 0.809, which is very close to an ideal value and the quotient resulting from the division of one slope value by the other for the finely pulverized drum-dried product (A) is considered to be closer to the ideal value than that for the drum-dried product (B) because the extraction rate for A is much faster than that for B. Therefore, it is believed that the quotient resulting from dividing one slope value by the other (i.e., $b_{25}/b_{35}$) for A falls between 0.807 and 0.809.

[Table 1]

Table 1: Slope value (b) for the semilog plot of (1-E) vs. $t$

| T (°C) | Sample B | Sample C | Sample D |
|---|---|---|---|
| 25 | 0.00267 | 0.00137 | 0.00096 |
| 35 | 0.0033 | 0.00288 | 0.00192 |
| $b_{25}/b_{35}$* | 0.809 | 0.476 | 0.500 |
| *: The value of $b_{25}/b_{35}$ calculated based on the relationship "$D_\eta/T$= Constant" is 0.807. | | | |

A "special obstacle" with regard to the diffusion coefficient upon extraction, means that if a shell-like heterogeneous layer is present on the particle surface, then there will be resistance from the shell-like heterogeneous layer when the substance being extracted (such as astaxanthin) passes through said shell. On the other hand, (there being) no special obstacle means that the particle surface is porous and there is no heterogeneous layer on the solid-liquid interface. The results shown in Figs. 3 to 5 and table 1 revealed that there were no special obstacles for sample B, while on the other hand, there were obstacles for samples C and D. This means that sample B differs from samples C and D in terms of physical properties. In addition, the extraction rate for sample A was extremely high and since sample A was obtained by further finely pulverizing sample B, it is highly probable that there is no special obstacle regarding the diffusion coefficient upon extraction for sample A, as is the case for sample B. Therefore, sample A is also considered to differ from samples C and D in terms of physical properties.

Observation via electron microscopy revealed that the dried bacterial cell powder of the drum-dried product (B) and that of the finely pulverized product (A) of the present invention both have sheet-like or solidified gel-like shapes. The physical properties of B are such that there are no special obstacles in the process of carotenoid diffusion upon extraction as described above. That is, the particles are porous or do not form heterogeneous layers and, therefore, can be readily digested and absorbed *in vivo.* It is believed that the same applies to product A. This is also supported by the fact that the *in vivo* absorption of astaxanthin contained in a finely powderized product is excellent, as shown in Fig. 1. On the other hand, as described above, there were special obstacles in the process of carotenoid diffusion upon extraction regarding C and D. The cause of this is believed to be because the particles of the spray-dried products (C and D) were found to predominantly have spherical shapes when observed via electron microscopy, and were rapidly spray-dried over a short period of time, which may result in rapid shrinkage of particle surface pores, in turn leading to surface hardening. As a result, it is believed that the *in vivo* digestion of such particles would be delayed, making it more difficult for carotenoids (e.g., astaxanthin) to be absorbed *in vivo* than would be the case with drum-dried products.

Example 3: Chicken egg yolk color enhancement (correlation between the particle size of dried bacterial cell powder and yolk color enhancement)

A drum-dried bacterial cell product was pulverized using a Jet Mill (Seishin Enterprise Co., Ltd.), thereby obtaining several finely pulverized drum-dried products having different average particle sizes.
The obtained dried bacterial cells were added to a basal feed (Multilayer RG, Chubushiryo Co., Ltd.) so as to obtain feeds having astaxanthin concentrations of 0.4 mg/100 g and 0.8 mg/100 g.
Laying hens were divided into 10 groups (of 5 individuals each) and raised for 4 weeks. See Table 2. The feed dosage was set to 100 g/day.

[Table 2]

Table 2: Test group

| Test group | Sample | Concentration of pigment added [mg/100 g] | |
|---|---|---|---|
| 1 | Finely pulverized drum-dried product: 7 $\mu$m | Astaxanthin | 0.4 |
| 2 | | | 0.8 |
| 3 | Finely pulverized drum-dried product: 9 $\mu$m | | 0.4 |
| 4 | | | 0.8 |
| 5 | Finely pulverized drum-dried product: 12 $\mu$m | | 0.4 |
| 6 | | | 0.8 |
| 7 | Finely pulverized drum-dried product: 20 $\mu$m | | 0.4 |
| 8 | | | 0.8 |
| 9 | Drum-dried product: 100 $\mu$m | | 0.4 |
| 10 | | | 0.8 |

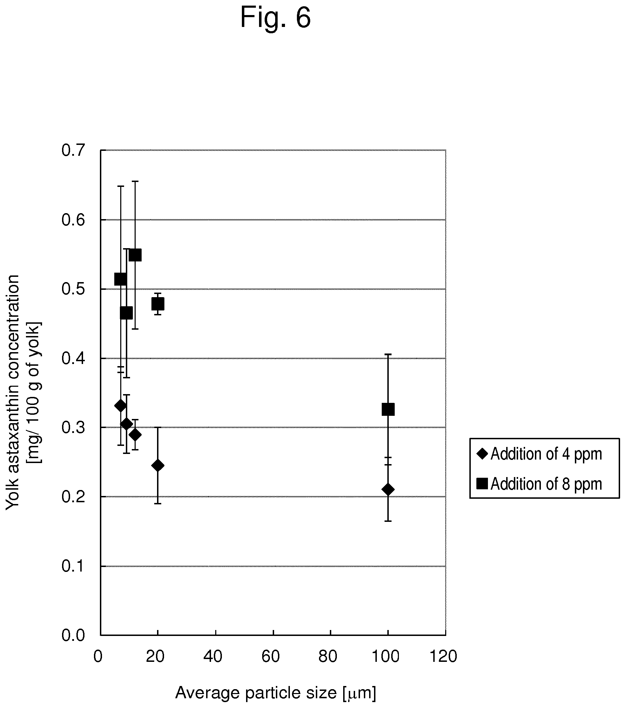

After feeding for 4 weeks, eggs were collected from each experimental group. The content of astaxanthin in the obtained chicken egg yolk was determined by high performance liquid chromatography. Results are shown in Fig. 6.

Industrial Applicability

A dried bacterial cell powder containing a carotenoid obtained by the production method of the present invention can be used in feeds for poultry farming to enhance the yolk color for poultry.

**Claims**

1. A method for producing a dried bacterial cell powder containing astaxanthin, said method comprising the step of bringing bacterial cells of an astaxanthin-producing microorganism of the genus Paracoccus into contact with a heat transfer unit having a temperature of more than 100°C to carry out drying via heat transfer and the step of further pulverizing the dried bacterial cell powder for fine powderization, wherein the volume particle size (D50) of the dried bacterial cell powder is pulverized to 20 $\mu$m or less by the pulverization step.

2. The production method according to claim 1, wherein the temperature of the heat transfer unit is equal to or exceeds 120°C.

3. The production method according to claim 1 or 2, wherein the drying is drying carried out by using a drum dryer, drying carried out by using a vacuum box dryer, drying carried out by using a multi-cylinder dryer, drying carried out by using a trough dryer, drying carried out by using a shelf dryer, or drying carried out by using a hot plate dryer.

4. The production method according to any one of claims 1 to 3, wherein the produced powder is a powder for being added to a feed for poultry.

5. A method for producing a feed for poultry, said method comprising the step of adding a dried bacterial cell powder containing astaxanthin produced by the method according to any one of claims 1 to 4 to a feed for poultry.

6. A dried bacterial cell powder containing astaxanthin produced by the method according to any one of claims 1 to 4.

7. Use of a dried bacterial cell powder containing astaxanthin produced by the method according to any one of claims 1 to 4 for the manufacture of feed for poultry.

**Patentansprüche**

1. Verfahren zur Herstellung von einem Astaxanthin enthaltenden Pulver getrockneter bakterieller Zellen, wobei das Verfahren den Schritt des Inkontaktbringens von bakteriellen Zellen eines Astaxanthin produzierenden Mikroorganismus der Gattung Paracoccus mit einer Wärmeübertragungseinheit mit einer Temperatur von mehr als 100 °C zur Ausführung der Trocknung durch Wärmeübertragung und den Schritt des weiteren Pulverisierens des Pulvers getrockneter bakterieller Zellen zur Feinpulverisierung umfasst, wobei die volumenbezogene Teilchengröße (D50) des Pulvers getrockneter bakterieller Zellen durch den Pulverisierungsschritt auf 20 μm oder weniger pulverisiert wird.

2. Herstellungsverfahren nach Anspruch 1, wobei die Temperatur der Wärmeübertragungseinheit 120 C entspricht oder diese übersteigt.

3. Herstellungsverfahren nach Anspruch 1 oder 2, wobei es sich bei dem Trocknen um Trocknen, das unter Verwendung eines Trommeltrockners, Trocknen, das unter Verwendung eines Vakuumschranktrockners, Trocknen, das unter Verwendung eines Mehrzylinder-Trockners, Trocknen, das unter Verwendung eines Muldentrockners, Trocknen, das unter Verwendung eines Trockenschranks oder Trocknen, das unter Verwendung eines Heizplatten-Trockners ausgeführt wird, handelt.

4. Herstellungsverfahren nach einem der Ansprüche 1 bis 3, wobei es sich bei dem hergestellten Pulver um ein Pulver zur Zugabe zu einem Futter für Geflügel handelt.

5. Verfahren zur Herstellung eines Futters für Geflügel, wobei das Verfahren den Schritt des Zugebens eines Astaxanthin enthaltenden Pulvers getrockneter bakterieller Zellen, das durch das Verfahren nach einem der Ansprüche 1 bis 4 hergestellt wurde, zu einem Futter für Geflügel umfasst.

6. Astaxanthin enthaltendes Pulver getrockneter bakterieller Zellen, das nach einem der Ansprüche 1 bis 4 hergestellt wurde.

7. Verwendung eines Astaxanthin enthaltenden Pulvers getrockneter bakterieller Zellen, das durch das Verfahren nach einem der Ansprüche 1 bis 4 hergestellt wurde, zur Erzeugung eines Futters für Geflügel.

**Revendications**

1. Procédé pour la production d'une poudre de cellules bactériennes séchées contenant de l'astaxanthine, ledit procédé comprenant l'étape de mise en contact de cellules bactériennes d'un micro-organisme produisant de l'astaxanthine du genre Paracoccus avec une unité de transfert de chaleur possédant une température supérieure à 100 °C pour mettre en œuvre un séchage via transfert de chaleur et l'étape en outre de pulvérisation de la poudre de cellules bactériennes séchées pour une pulvérisation fine, la grosseur de particule en volume (D50) de la poudre de cellules bactériennes séchées étant pulvérisée à 20 μm ou moins par l'étape de pulvérisation.

2. Procédé de production selon la revendication 1, la température de l'unité de transfert de chaleur étant égale ou excédant 120 °C.

3. Procédé de production selon la revendication 1 ou 2, le séchage étant un séchage mis en œuvre à l'aide d'un séchoir à tambour, un séchage mis en œuvre à l'aide d'un séchoir à boîte à vide, un séchage mis en œuvre à l'aide d'un séchoir à plusieurs cylindres, un séchage mis en œuvre à l'aide d'un séchoir traversant, un séchage mis en œuvre à l'aide d'un séchoir à étages, ou un séchage mis en œuvre à l'aide d'un séchoir à plaque chaude.

4. Procédé de production selon l'une quelconque des revendications 1 à 3, la poudre produite étant une poudre destinée à être ajoutée à un aliment pour volaille.

5. Procédé pour la production d'un aliment pour volaille, ledit procédé comprenant l'étape d'ajout d'une poudre de cellules bactériennes séchées contenant de l'astaxanthine produite par le procédé selon l'une quelconque des revendications 1 à 4 à un aliment pour volaille.

6. Poudre de cellules bactériennes séchées contenant de l'astaxanthine produite par le procédé selon l'une quelconque des revendications 1 à 4.

7. Utilisation d'une poudre de cellules bactériennes séchées contenant de l'astaxanthine produite par le procédé selon l'une quelconque des revendications 1 à 4 pour la fabrication d'aliments pour volaille.

# Fig. 1

# Fig. 2

# Fig. 3

# Fig. 4

# Fig. 5

$\ln y = \ln(1.025) - 0.00096x$
$R^2 = 0.9328$

$\ln y = \ln(0.950) - 0.00191x$
$R^2 = 0.9287$

□ Granulated spray-dried product (25°C)

◇ Granulated spray-dried product (35°C)

1-E

1.00

0.10

0.00    100.00    200.00    300.00    400.00

Time [minutes]

# Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2554675 A1
- US 2013330298 A1
- JP 2006101721 A
- JP 2006340676 A
- JP H8182 A
- JP 2001095500 A
- JP 2003304875 A
- JP 2005087097 A
- JP 2005087100 A

### Non-patent literature cited in the description

- **AN, G-H. et al.** Improved Astaxanthin availability due to drying and rupturing of the red yeast, Xanthophyllomyces dendrorhous. *FOOD SCI. BIOTECHNOL.,* 2006, vol. 15 (4), 506-510
- *Seibutsu-kogaku Kaishi (Bioengineering),* 2010, vol. 88 (9), 492-493